# EUROPÄISCHE PATENTSCHRIFT

(11) **EP 1 171 176 B1**
(45) Veröffentlichungstag und Bekanntmachung des Hinweises auf die Patenterteilung: **19.03.2003**
(21) Anmeldenummer: 00934922.6
(22) Anmeldetag: 20.04.2000
(51) Int. Cl.: A61L 27/26, A61L 27/34, A61L 27/22, A61L 27/48, A61M 31/00, A61F 2/06, A61F 2/44

(54) **IMPLANTAT FÜR DIE WIEDERHERSTELLUNG VON WIRBELN UND RÖHRENKNOCHEN**
IMPLANT FOR RECREATING VERTEBRAE AND TUBULAR BONES
IMPLANT POUR LA RECONSTITUTION DE VERTEBRES ET D'OS TUBULAIRES

(30) Priorität: 20.04.1999 DE 19917696
(43) Veröffentlichungstag der Anmeldung: 16.01.2002
(73) Patentinhaber: SCHMIDT, Karlheinz, Prof. Dr. Dr., 72810 Gomaringen (DE)
(72) Erfinder: SCHMIDT, Karlheinz, Prof. Dr. Dr., 72810 Gomaringen (DE)
(74) Vertreter: Castell, Klaus, Dr.-Ing.
(86) Internationale Anmeldenummer: DE0001280
(87) Internationale Veröffentlichungsnummer: WO00062835

(56) Entgegenhaltungen:
- WO-A-91/06324
- WO-A-93/20857
- US-A- 4 919 666
- US-A- 5 545 208

## Beschreibung

Die Erfindung betrifft ein Implantat, das einen Wirkstoffkomplex aufweist, mit den folgenden, voneinander unterschiedlichen Komponenten in Form mindestens einer Strukturkomponente, mindestens einer Rekrutierungskomponente, mindestens einer Adhäsionskomponente und mindestens einer Wachstums- und/oder Maturationskomponente.

Im Stand der Technik ist bereits ein Wirkstoffkomplex für die Herstellung von biologischen Teilen, insbesondere von Organen für Lebewesen, mit den genannten Komponenten bekannt. Bei diesem bekannten Wirkstoffkomplex kann die Strukturkomponente beispielsweise aus verschiedenen Collagenen, Elastin oder Proteoglycanen bestehen. Als Rekrutierungskomponente für diesen Wirkstoffkomplex sind insbesondere Chemotaktica zu nennen, beispielsweise Peptide, wie N-F-Met-Leu-Phe- und/oder beispielsweise Metabolite der Arachidonsäure, wie Leukotriene. Die Aufgabe der Adhäsionskomponente können Eiweißkörper vom Typ des Fibronectins oder Laminins, aber auch Zell-Adhäsions-Moleküle, wie L-CAM, N-CAM und Matrix-Adhäsions-Moleküle, wie Cytotactin, Tenascin, Collagen Typ IV, V, VII, synthetische Peptide und Transmembran-Verbindungsproteine, wie Integrin, erfüllen. Die zunächst genannten Beispiele für Adhäsionskomponenten, Fibronectin und Laminin, sind für die Zwecke des hier erläuterten Wirkstoffkomplexes in den Bereich der Matrix-Adhäsions-Moleküle einzuordnen. Als weitere Komponente weist der genannte Wirkstoffkomplex mindestens eine Wachstums- und/oder Maturationskomponente auf, vorzugsweise in Form eines oder mehrerer Cytokine. Beispiele für solche Cytokine sind bei der Herstellung von Blut die Kolonie-stimulierenden Faktoren, bei der Herstellung von Bindegewebe der Fibroblasten Wachstumsfaktor, bei der Herstellung von Haut der epidermale Wachstumsfaktor, bei der Herstellung von Knorpel der Knorpel induzierende Faktor, bei der Herstellung der Milz oder der Lymphknoten der Lymphocyten aktivierende Faktor sowie Milzpeptide, für die Herstellung von Thymus der T-Zellen Wachstumsfaktor sowie Thymuspeptide, für die Herstellung von Knochen der Knochen-Wachstumsfaktor sowie der transformierende Wachstumsfaktor, für die Herstellung von Blutgefäßen der Angiogenese-Faktor. Ferner finden noch die folgenden Cytokine Verwendung: Interleukine, Insulin-ähnliche Wachstumsfaktoren, der Tumor-Nekrose-Faktor, Prostaglandine, Leukotriene, transformierende Wachstumsfaktoren, von Thrombocyten abstammender Wachstumsfaktor, Interferone sowie von Endothelzellen abstammender Wachstumsfaktor.

Näheres über diesen Wirkstoffkomplex ist aus dem europäischen Patent Nr. 0 500 556 zu entnehmen, dessen Offenbarungsgehalt hier ausdrücklich einbezogen wird.

Dieser Wirkstoffkomplex hat nach seiner Herstellung zunächst eine watteartige Konsistenz. Wenn größere Knochendefekte auszufüllen sind, muß der als Implantat eingebrachte Wirkstoffkomplex eine ausreichende Eigenfestigkeit haben, um durch die umgebenden Weichgewebe oder Knochenstruren nicht komprimiert zu werden. Er muß daher vor der genannten Anwendung bereits komprimiert werden, was zu einer höheren mechanischen Festigkeit, aber auch zu einem hohen Materialverbrauch führt oder es muß ein ausreichend stabiles Trägermaterial zusammen mit dem Wirkstoffkomplex eingesetzt werden. Die Kombination eines Trägermaterials mit dem Wirkstoffkomplex ist jedoch keinesfalls unproblematisch. Nach den bisher zur Verfügung stehenden Erkenntnissen über den Wirkstoffkomplex und seine komplexe Wirkungsweise ist zumindest eine Beeinträchtigung der Bildung oder Wiederherstellung des jeweiligen zu behandelnden biologischen Teils, z. B. der knöchernen Regeneration, zu befürchten. Auch die Gefahr einer gewebetoxischen Reaktion wurde vermutet.
Außerdem sind Anwendungen des Wirkstoffkomplexes bei Krankheiten oder Defekten bisher nicht möglich, bei denen das aus dem Wirkstoffkomplex bestehende Implantat so hohen mechanischen Belastungen ausgesetzt wird, daß auch die mechanische Festigkeit eines komprimierten Materials nicht ausreichen kann.

Davon ausgehend lag der vorliegenden Erfindung daher die Aufgabe zugrunde, ein Implantat bereitzustellen, mit welchem eine hohe mechanische Festigkeit erreicht wird, um so die Einsatzmöglichkeiten des Wirkstoffkomplexes zu erweitern.

Gelöst wird diese Aufgabe durch ein Implantat, bei dem ein metallischer, nichtmetallischer oder keramischer Hohlkörper mit dem genannten Wirkstoffkomplex beschichtet ist oder diesen Wirkstoffkomplex aufweist, wodurch ein Implantat entsteht, das für die zumindest teilweise Herstellung, Wiederherstellung oder Stabilisierung von Wirbelkörpern oder Röhrenknochen verwendet werden kann. Die Komponenten des Wirkstoffkomplexes sind dabei auf die Herstellung von Knochen abgestimmt, wozu auch gehört, daß sich alle für die Versorgung des Knochens bzw. Wirbels notwendigen Strukturen, wie z. B. Blutgefässe und Nerven bilden.

Die Lösung der Aufgabe lag schon deshalb nicht nahe, weil es, wie bereits erläutert, durchaus problematisch ist, eine Kombination des Wirkstoffkomplexes mit einem Träger, mit dem der Wirkstoffkomplex beschichtet ist oder den er aufweist, vorzusehen, weil dann die Funktionen des Wirkstoffkomplexes, beispielsweise in dem Knochendefekt, gestört sein oder zumindest durch mögliche Immunreaktionen kompliziert werden könnten.

Der metallische Hohlkörper besteht vorzugsweise aus Titan, auch in Form von Titan-Legierungen. Dabei wurde insbesondere eine Legierung aus den Komponenten Titan, Aluminium und Vanadin untersucht. In einer bevorzugten Ausführungsform werden die metallischen Träger in Form von zylindrischen Hohlkörpern mit Gitterstruktur eingesetzt.

Als nichtmetallisches Material ist vor allen Dingen Kohlenstoff zu nennen, der in Form sogenannter "Carbon-Käfige" oder "carbon cages", bestehend aus Kohlenstoff-Fasern, eingesetzt werden kann, welche ebenfalls zylindrische Hohlkörper bilden können. Sowohl die Titan-(Hohl-) Körper als auch die Carbon-Käfige werden mit dem Wirkstoffkomplex befüllt oder auf ihrer nach innen weisenden Fläche mit dem Wirkstoffkomplex beschichtet.

Die genannten Titanhohlkörper und Carbon-Käfige können, wenn sie mit dem Wirkstoffkomplex befüllt oder beschichtet worden sind, zur Herstellung, Wiederherstellung oder Stabilisierung von Wirbelkörpern verwendet werden. Damit ergibt sich die einzigartige Möglichkeit, Defekte an Wirbeln oder zerstörte Wirbel der Wirbelsäule durch Verblockung von Wirbelkörpern und vollständiger Regeneration des Wirbels wiederherzustellen.

Eine Verblockung von Wirbelkörpern ist häufig erforderlich, wenn durch degenerative Prozesse an den Bandscheiben, Tumoren oder Metastasen in den Wirbelkörpern der Wirbelsäule oder auch durch Osteoporose die Tragfähigkeit der Wirbelsäule beeinträchtigt ist, so daß entweder Wirbelfrakturen oder Nervenläsionen drohen. In diesen Fällen ist es erforderlich, die Kontinuität der Wirbelsäule über ein mechanisch stabiles Implantat, wie den Titankörper oder Carbon-Käfig zu sichern. Die erforderliche knöcherne. Überbrückung konnte bislang nur durch in einem Zweiteingriff gewonnene autologe Spongiosa, z. B. Spongiosa aus dem Beckenkamm, versucht werden, was eine Reihe von Problemen beinhaltet, wie z. B. den Zweiteingriff und das damit verbundene Operationsrisiko mit einer zusätzlichen Infektionsgefahr, die begrenzte Menge an gewinnbarer Spongiosa und Komplikationen an der Entnahmestelle, wie Infektionen oder chronische Schmerzzustände. Auch die Verfügbarkeit solcher Autotransplantate ist beschränkt.

Durch die Befüllung oder Beschichtung der Titanhohlkörper oder Carbon-Käfige mit dem Wirkstoffkomplex konnte in kurzer Zeit eine knöcherne Überbrückung erreicht werden, ohne autologe Spongiosa zu benötigen. Die Gitterstruktur des Titan-Hohlkörpers erlaubte eine rasche Vaskularisierung auch im Inneren der formstabilen Komponente, so daß der Wirkstoffkomplex seine Aktivität entfalten kann und die Knochenbildung über das gesamte erforderliche Volumen eintritt, ohne das mechanische Kräfte die Form des neugebildeten Knochen beeinträchtigen. Neben der Verwendung im Bereich der Wirbelkörper kann ein solcher Titanhohlkörper oder Carbon-Käfig sowie die nachfolgend beschriebenen keramischen Hohlkörper auch an beliebigen anderen Implantationsorten verwendet werden, wie z. B. im Kieferbereich, an Röhrenknochen und grundsätzlich bei der Augmentation von Knochenmasse.

Mit dem bisherigen Wirkstoffkomplex war die Verblockung von Wirbeln deshalb nicht möglich, weil dieser der mechanischen Belastung innerhalb der Wirbelsäule nicht Stand gehalten hätte. Die nun mit dem Wirkstoffkomplex befüllten oder beschichteten Hohlkörper oder Käfige schaffen die mechanische Stabilität, ohne jedoch immunologische Gegenreaktionen oder eine verschlechterte Wirksamkeit des Wirkstoffkomplexes nach sich zu ziehen.

Neben metallischen oder nichtmetallischen Hohlkörpern sind auch Hohlkörper aus keramischen Materialien einsetzbar. Unter den keramischen Trägermaterialien sind insbesondere Glaskeramiken zu nennen, wie Calciumphosphatkeramiken, Aluminiumoxidkeramiken und Hydroxylapatitkeramiken.

Die Calciumphosphatkeramiken basieren auf dem System CaO/P₂O₅. Auf der Basis dieses Systems existieren fünf verschiedene binäre Verbindungen. Dabei haben sich Tricalciumphosphat (TCP) und Tetracalciumphosphat für die Zwecke der vorliegenden Erfindung als geeignet erwiesen.

TCP wird durch Pressen und anschließendes Sintern der Ausgangsmaterialien Calciumoxid (CaO) und Diphosphorpentoxid (P₂O₅) hergestellt. Alternativ kann es auch in einem Arbeitsschritt durch Heißpressen hergestellt werden.

Tetracalciumphosphat wird ähnlich wie TCP in zwei Schritten hergestellt, indem zunächst die Ausgangsstoffe auf Kristallgitterabstände von 5 bis 10 µm verdichtet und die Masse dann bei 1100 bis 1500°C gebrannt wird.

Hydroxylapatit wird durch keramisches Brennen von Pentacalciumhydroxid-Triphosphatpulver bei 1250°C gewonnen. Außerdem kann zur Herstellung einer Hydroxylapatitkeramik auch ein natürliches Material herangezogen werden, wie das carbonatische Skelett der Rotalge. Dabei werden nach einem Wasch- und Trocknungsvorgang zunächst die organischen Bestandteile durch Pyrolyse bei einer Temperatur von etwa 700°C entfernt. Dann erfolgt die Umsetzung zu Hydroxylapatit unter Hinzufügung von Phosphatlösung bei erhöhtem Druck und erhöhter Temperatur.

Bei einem weiteren Herstellungsverfahren einer Hydroxylapatitkeramik, ausgehend von dem natürlichen Skelett von Korallen, wird das Calciumcarbonat der Korallen durch hydrotermale Umwandlung in Hydroxylapatit oder eine Mischung aus Hydroxylapatit und weiteren mineralischen Strukturen umgewandelt. Bei dem so entstehenden Material bleibt die koralline Struktur, d. h. insbesondere das interkonnektierende Porensystem der Koralle, erhalten.

Aluminiumoxidkeramiken, die polykristallin aufgebaut sind, enthalten zu etwa 99,7 % Aluminiumoxid sowie geringe Anteile an Magnesiumoxid und/oder Zirkonoxid. Sie werden nach Vorverdichtung unter hohem Druck bei Temperaturen von etwa 1500 bis 1800°C zu einem Festkörper gesintert. Für die Zwecke der vorliegenden Erfindung wurden mikroporöse Aluminiumoxidkeramiken verwendet. Auch monokristalline Formen (Saphire) können zur Anwendung kommen.

Der Wirkstoffkomplex selbst kann zusätzlich auf Trägermaterialien aufgebracht sein, die ausgewählt sind aus Polymeren und Collagenen. Damit kann die Menge an zur Auffüllung des jeweiligen Hohlkörpers benötigtem Wirkstoffkomplex reduziert werden, um die Kosten zu verringern, bei im wesentlichen gleichbleibender knochenbildender Wirksamkeit.

Bei den polymeren Trägermaterialien kommen insbesondere Polymere aus natürlichen Monomeren, wie Polyaminosäuren (Polylysin, Polyglutaminsäure, etc.) und Polymere der Milchsäure in Betracht. Es können auch Copolymere, z. B. aus Polymilchsäure und Hydroxyessigsäure, verwendet werden.
Polylaktate sind Polyester der Milchsäure mit der chemischen Formel:

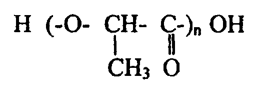

Bei der direkten Polymerisation der Monomere ergeben sich Polymere mit relativ niedrigen Molekulargewichten. Die obere Grenze liegt etwa bei 20000 Da. Höhere Molekulargewichte können durch die Verknüpfung zyklischer Dimere bei hoher Temperatur, geringem Druck und in Gegenwart von Katalysatoren entstehen. Die Milchsäurepolymere sind bioabbaubar, biokompatibel, wasserunlöslich und zeichnen sich durch eine große Festigkeit aus.

Des weiteren können verschiedene Collagene als Trägermaterial verwendet werden. Hierbei sind insbesondere die Collagene vom Typ I, IV, V, VII zu nennen. Die Collagene können beispielsweise in Form von Vliesen oder Gelen eingesetzt werden und weisen insbesondere eine an sich gute immunologische Verträglichkeit in Verbindung mit einer problemlosen Verarbeitung auf.

Im folgenden wird die Erfindung anhand von Beispielen und unter Bezugnahme auf die beigefügte Zeichnung näher erläutert.

Es zeigen:
- Fig. 1:: eine schematische Darstellung der Knochenneubildung bei Kaninchen unter Verwendung des Wirkstoffkomplexes, im Vergleich zu einer Leerprobe,
- Fig. 2:: eine schematische Darstellung der Knochenneubildung bei Schafen unter Verwendung des Wirkstoffkomplexes, mit Tricalciumphosphat als Trägermaterial, im Vergleich zu reinem Tricalciumphosphat,
- Fig. 3:: eine schematische Darstellung der Knochenneubildung bei Ratten unter Verwendung des Wirkstoffkomplexes, mit verschiedenen Collagenen als Trägermaterial, im Vergleich zu den reinen Collagenen,
- Fig. 4:: eine schematische Darstellung eines Hohlkörpers aus KohIenstoff-Fasern ohne Gitterstruktur, mit zwei Kammern fiir den Vergleich zwischen Wirkstoffkomplex und autologer Spongiosa,
- Fig. 5a-c:: eine schematische Darstellung eines Hohlkörpers aus Titan, mit Gitterstruktur, der mit dem Wirkstoffkomplex befüllt ist und zur Verblockung von Wirbelkörpern dient,
- Fig. 6: die Darstellung eines Käfig- oder cage-Setzgerätes, das einen Carbon-Käfig mit zwei Kammern aufweist,
- Fig. 7a,b:: röntgenographische Darstellungen eines stark verringerten Lendenwirbelabstandes im Segment L5/S1 vor der Operation,
- Fig. 8a,b:: eine röntgenographische Darstellung eines Implantats zwischen den Wirbeln L4 und L5 der Lendenwirbelsäule mit dem zur Stabilisierung eingebrachten Fixateur intern,
und
- Fig. 9:: eine Abbildung einer mittels Computertomographie erhaltenen Bildsequenz, jeweils drei, sechs und neun Wochen nach dem Einbringen eines Carbon-Käfig-Implantats, mit Wirkstoffkomplex und autologer Spongiosa.

### 1. Herstellung des Wirkstoffkomplexes

Im folgenden werden die wesentlichen Herstellungsschritte des Wirkstoffkomplexes beschrieben:
Röhrenknochen von Kälbern, Schafen, Kaninchen oder Ratten wurden gesäubert und unter anderem vom Knochenmark befreit und dann eingefroren. Der gefrorene Knochen wird auf eine Partikelgröße von weniger als 2 mm zerkleinert. Die zerkleinerten Knochenstücke wurden in Aceton entfettet und in 0.6 N Salzsäure entkalkt. Danach wird lyophilisiert und man erhält eine demineralisierte Knochenmatrix, die in 4 molarer Guanidinium-HCl-Lösung extrahiert wird. Die Extraktionslösung wird gegen Aqua dest. dialysiert und der Wirkstoffkomplex durch Abzentrifugieren und Lyophilisieren im Präzipitat erhalten.

Diese grundsätzliche Herstellungsweise ist im nachfolgenden Flußdiagramm nochmals dargestellt.

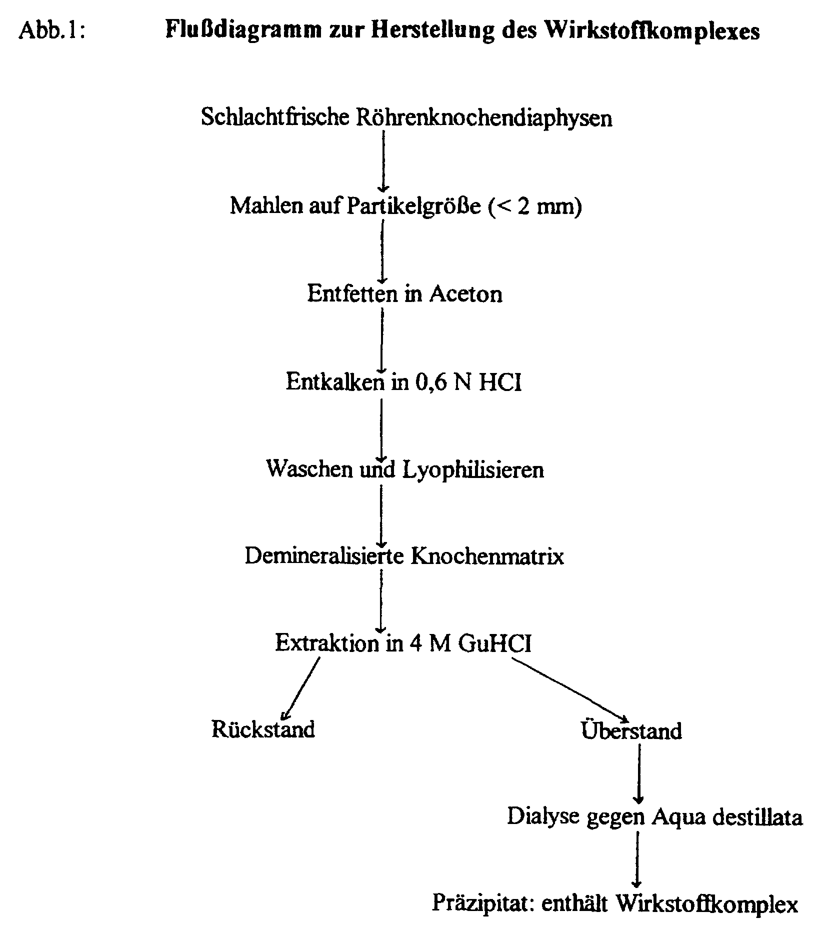

### II. Wirksamkeit des Wirkstoffkomplexes ohne Verwendung von Trägermaterialien

Um zu zeigen, daß der Wirkstoffkomplex als solches wirksam ist, wird zunächst ein Versuch dargestellt, bei dem der Wirkstoffkomplex ohne weitere Träger oder Trägermaterialien implantiert wird.

### 1. Für den Versuch verwendete Tiere

Es werden weibliche Kaninchen der Rasse Chinchilla mit einem mittleren Körpergewicht von 3089 g verwendet. Sie erhielten Haltungsfutter für Kaninchen und mit Salzsäure auf pH 4,5 angesäuertes doppelt ozoniertes Leitungswasser nach Bedarf.

Die Tiere wurden durch Subcutaninjektion eines Gemisches von Ketamin und Xylazin narkotisiert.

### 2. Vorbereitung eines Knochendefektes bei den Kaninchen

Mit einem innen gekühlten Bohrer wurde ein Implantatlager von 4 mm Durchmesser und cirka 9 mm Tiefe im Kniegelenk (distales Femurende) des Kaninchens präpariert. Dann wurde das so gebildete Bohrloch jeweils mit 30 und 90 mg des Wirkstoffkomplexes gefüllt, der hergestellt worden war, wie unter I. beschrieben. Jeweils ein weiteres Bohrloch diente in Form eines "Leerloches" zur Kontrolle der Knochenneubildung.

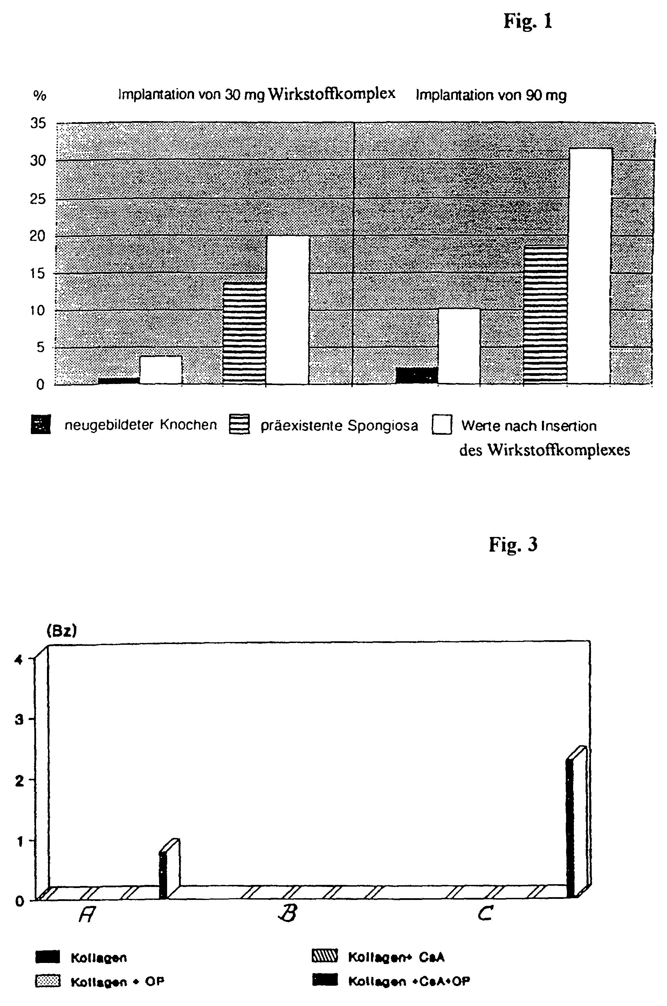

Fig. 1 zeigt die Knochenneubildung im Leerloch und im Bohrloch nach Implantation des Wirkstoffkomplexes sowie die Dichte der umgebenden präexistenten Spongiosa jeweils 28 Tage nach der Operation (n=2/Wirkstoff-Menge).

Bei der Auswertung der Versuche wurde festgestellt, daß die Dichte der die Bohrlöcher umgebenden Spongiosa nach Implantation von 30 mg des Wirkstoffkomplexes um 45 % und nach Implantation von 90 mg des Wirkstoffkomplexes um 69 % höher lag als beim Leerloch. Dabei hatte die Menge an präexistenter Spongiosa keinerlei Einfluß auf die Regeneration im Defekt, da die Knochenneubildung nach der Insertion des Wirkstoffkomplexes nicht von der Bohrlochperipherie ausging, sondern gleichmäßig über den Defekt verteilt war.

### III. Knochenbildung im Unterkiefer von Schafen unter Verwendung von Tricalciumphosphat (TCP)

### 1. Für die Versuche verwendete Tiere

Für die nachfolgend beschriebenen Untersuchungen wurden ausgewachsene Hausschafe von der Viehzentrale Südwest AG Stuttgart verwendet. Diese erhielten Heu und Wasser als Ernährung sowie drei Tage vor dem operativen Eingriff einen Brei aus Altromin-Pellets.

Die Tiere wurden mit 1 ml Xylazin/1ml Ketanest i. m. prämediziert. Dann wurden die Schafe mit Nembutal narkotisiert.

### 2. Vorbereitung des Implantats

TCP wurde in einer Lösung von 100 mg gelöstem Wirkstoffkomplex mit 10 ml Wasser suspendiert und unter ständigem Rühren mit flüssigem Stickstoff tiefgefroren. Nach 24-stündigem Lyophilisieren und anschließender Gassterilisation (Ethylenoxid) wurde das so mit dem Wirkstoffkomplex dotierte TCP in den nachfolgend beschriebenen Unterkiefer-Defekt eines Schafes eingebracht. Außerdem wurde ein weiterer Unterkiefer-Defekt, der als Vergleich diente, mit undotiertem, im Autoclaven sterilisiertem TCP gefüllt.

### 3. Vorbereitung des Unterkiefer-Defektes beim Schaf

In einem entsprechend vorbereiteten Unterkiefer eines Schafes wurde unter Kühlung mit physiologischer Kochsalzlösung mittels eines Trepanbohrers von 5 mm Durchmesser jeweils ein normierter Knochenzylinder herausgefräßt und entfernt. Dann wurde eines der so gebildeten Bohrlöcher mit TCP, das gemäß der Versuchsvorschrift 1. mit dem Wirkstoffkomplex dotiert worden war, befüllt und das zweite Bohrloch mit undotiertem TCP aufgefüllt.

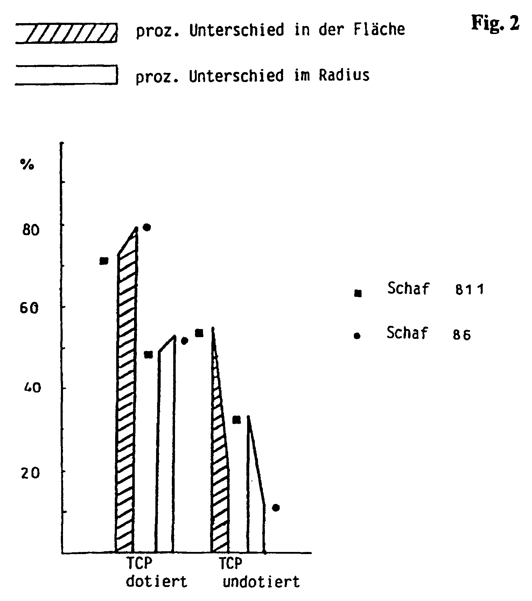

Die Ergebnisse des Knochenwachstums in den Unterkieferdefekten sind in Fig. 2 zur Erleichterung der Übersicht grafisch dargestellt. Die Versuchsdauer betrug 26 bzw. 41 Tage.

Es zeigte sich, daß durch die Dotierung von TCP mit dem Wirkstoffkomplex eine Beschleunigung der knöchernen Regeneration des Unterkieferdefektes der beiden Schafe Nr. 811 und 86 in der Anfangsphase von etwa 100 % erreicht wurde. Nach 41 Tagen betrug die Steigerung der Beschleunigung der knöchernen Regeneration immer noch 10 %. Die Knochenheilung verläuft daher besonders am Beginn deutlich schneller als ohne die osteoproduktive Wirkung der mit dem Wirkstoffkomplex dotierten Implantate.

### IV. Versuche mit Collagenen als Trägermaterialien

Bei der Herstellung des Wirkstoffkomplexes ist der mengenmäßige Ertrag in dem erforderlichen Reinheitsgrad sehr gering. Daher wurde untersucht, ob es Trägermaterialien gibt, die mit dem Wirkstoffkomplex kombiniert werden können, um so die Menge des fiir die jeweilige Zielsetzung benötigten Wirkstoffkomplexes reduzieren zu können, ohne dadurch seine knochenbildende Wirksamkeit zu verringern.

### 1. Wirkstoffkomplex

Der für die Zwecke der nachfolgend beschriebenen Versuche verwendete Wirkstoffkomplex wurde genauso hergestellt wie unter **I.** beschrieben, wobei Röhrenknochen von Kälbern verwendet wurden.

### 2. Versuchstiere

Es wurden männliche Wistarratten eines Gewichtes zwischen 350 und 400 g verwendet und in einem klimatisierten Tierstall bei 23° C und etwa 50 % relativer Luftfeuchtigkeit gehalten. Ernährt wurden sie mit einer Haltungsdiät für Ratten und Mäuse.

Jedem untersuchtem Tier wurden zwei Implantate desselben Trägermaterials in die Bauchmuskulatur eingebracht, von denen das eine mit dem Wirkstoffkomplex beschichtet war, während das andere als Vergleichsimplantat unbeschichtet blieb. Die Tiere wurden nach 21 Tagen getötet und die betreffenden Areale der Implantate in der Bauchmuskulatur explantiert und histologisch ausgewertet.

### 3. Verwendete Trägermaterialien

Für diese Versuche wurden Collagenmaterialien eingesetzt, die alle käuflich zu erwerben sind. Collagen A war ein reines, steriles, natives, resorbierbares Rinderhautcollagen, das frei ist von jeglichen Fremdzusätzen, wie Stabilisatoren oder Desinfizienzien.
Collagen B war ein gereinigtes, lyophilisiertes, leicht quervemetztes steriles und nicht pyrogenes Rinderhautcollagen mit schwachen antigenen Eigenschaften. Die helikale Struktur des Collagens blieb erhalten.
Collagen C bestand aus reinen, nativen und resorbierbaren Rindercollagenfibrillen.

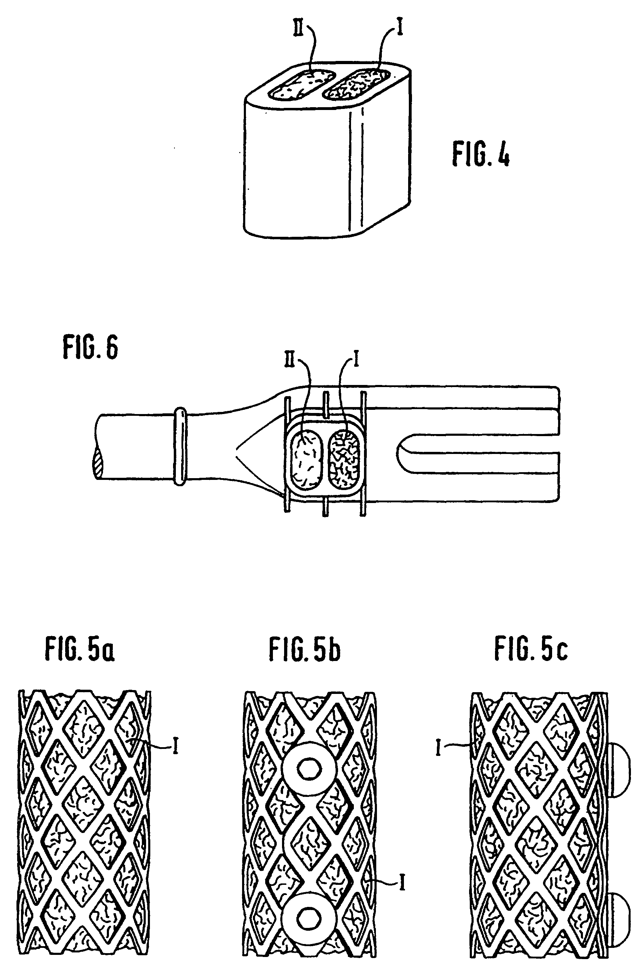

Alle verwendeten Collagene lagen in Vliesform vor. Es wurden Collagenvliesstücke von je 50 mg abgeschnitten und je 1 ml der Wirkstoffkomplex-Lösung (3mg/ml) zugegeben. Bei den Kontrollimplantaten wurde stattdessen 1 ml destillierten Wasser zugegeben. Die so behandelten Collagen - Vliesstücke wurden bei -20° C eingefroren, lyophilisiert und ergaben Implantate mit einem Durchmesser von etwa 10 mm und einer Dicke von etwa 5 mm. Fig. 3 zeigt die Ergebnisse der Knochenbildung der Collagenimplantate A, B und C in mit und ohne Beschichtung mit dem Wirkstoffkomplex (Cyclosporin A) immunsupprimierten Tieren als auch nicht immunsupprimierten Tieren nach 21 Tagen. Dabei entspricht die Bewertungszahl (BZ) dem arithmetischen Mittel der Bewertungszahlen von drei unabhängigen Personen bei sechs Implantaten jeder Gruppe.

Mit dem Wirkstoffkomplex beschichtetes Collagen A zeigte nach dieser Zeit bei immunsupprimierten Tieren einen knochenbildenden Effekt, während dieser bei Collagen B nicht nachgewiesen werden konnte. Demgegenüber zeigte jedoch Collagen C einen sehr ausgeprägten knochenbildenden Effekt.

Daraus folgt, daß es auf die Präparation des jeweils verwendeten Collagens ankommt und sich seine Eignung als Trägermaterial daraus ergibt. Collagene, die immunogen sind, sind zur Verwendung als Trägermaterialien ungeeignet.

### IV. Prüfung von metallischen und keramischen Materialien auf ihre Biokompatibilität

Es wurden Titanplättchen mit unterschiedlicher Rauhigkeit (100, 20 und 0,5 µm), eine TiAl₆V₄-Legierung (0,5µm) und Al₂O₃-Plättchen der Firma Friedrichsfeld sowie Hydroxylapatit-Plättchen von der Feldmühle AG eingesetzt.
Die Beschichtungen mit dem Wirkstoffkomplex, welcher nach dem weiter oben angegebenen allgemeinen Verfahrensschema unter Verwendung von Röhrenknochen von Kälbern hergestellt worden war, wurden durch das Beschichtungsverfahren "Dip-coating" aufgebracht. Unter "Dip-coating" wird ein Beschichtungsverfahren verstanden, bei dem der zu beschichtende Gegenstand, hier die Plättchen, in eine Lösung mit einer gewünschten, vorgegebenen Konzentration des Beschichtungsmittels, hier des Wirkstoffkomplexes, getaucht wird. Anschließend wird lyophilisiert. Es werden dünne Überzugsschichten bzw. Beschichtungen erhalten. Die Prüfung der angegebenen Materialien auf ihre Biokompatibilität wurde insbesondere in Bezug auf die Rauhigkeit der Oberflächen durchgeführt (n=20; je vier Plättchen). Tabelle 1 gibt die dabei erhaltenen Ergebnisse wieder.

Bei dieser Biokompaktibilitätsprüfung der untersuchten Materialien zeigte sich, daß Titan aufgrund der höchsten Anzahl lebender Zellen sowie den besten Verhältnis lebender : toter Zellen als Trägermaterial sehr geeignet ist. Während Hydroxylapatit ein ähnlich gutes Ergebnis lieferte, schnitt TiAl₆V₄ erheblich schlechter ab.

Generell zeigte sich in Bezug auf die Oberflächenrauhigkeiten, daß die glattesten Oberflächen, d. h. Oberflächen mit einem Porendurchmesser von 0,2 - 0,5 µm, mit Ausnahme von TiAl₆V₄ die besten Resultate lieferten. Mit Zunahme der Rauhigkeit bzw. des Porendurchmessers sinken sowohl die Anzahl lebender Zellen als auch das Verhältnis lebender : toter Zellen. Bei einem Porendurchmesser von etwa 0,5 µm wurde der höchste Anteil an lebendem (Knochen)-Gewebe in unmittelbarem Kontakt zur Plättchengrenzfläche erhalten.

**Tabelle 1:**

| Trägermaterial | Anzahl lebender Zellen/cm² | Anzahl toter Zellen/cm² |
|---|---|---|
| Hydroxylapatit | | |
| 0,2 - 0,5 µm | 1792 ± 700 | 200 ± 37 |
| 20 µm | 7469 ± 2614 | 2238 ± 715 |
| 50 µm | 4477 ± 408 | 1692 ± 427 |
| Osprovit (Feldmühle) | 7930 ± 2007 | 1638 ± 377 |
| Titan 0,5 µm | 11377 ± 2538 | 1054 ± 308 |
| 20 µm | 9600 ± 3038 | 1754 ± 439 |
| 100 µm | 2308 ± 669 | 2085 ± 623 |
| TiAl₆V₄ 0,5 µm | 7200 ± 1062 | 2800 ± 954 |
| Al₂O₃ reinst, poliert | 11446 ± 1500 | 2292 ± 600 |

### V. Titankörper und Carbonkäfige

Nachdem die unter **IV.** dargestellten Untersuchungen die grundsätzliche Biokompatibilität des Titans nachwiesen, ergab sich eine besondere Anwendungsmöglichkeit des Wirkstoffkomplexes in formstabilen Käfigen aus Titan zur Verblockung von Wirbelkörpern (Spondylodese). Außerdem zeigten sich hierfür auch Carbon-Käfige, sogenannte "carbon-cages" als geeignet.

Es konnte für den Fall der Spondylodese ein Eingriff an der Wirbelsäule eines Menschen durchgeführt und dokumentiert werden, der einen Vergleich zwischen der Verwendung autologer Spongiosa und dem Wirkstoffkomplex ermöglichte.

Hierfür wurde ein Carbon-Käfig ("carbon-cage") mit zwei Kammern verwendet. Ein solcher carbon-cage ist schematisch in Fig. 4 dargestellt. Anstelle des carbon-cage kann völlig gleichwertig ein Titan-Hohlkörper eingesetzt werden, der schematisch in den Fig. 5a, b, c dargestellt ist. Die Figuren 5a - 5c zeigen unterschiedliche Ansichten des mit dem Wirkstoffkomplex befüllten Titan-Hohlkörpers.
Für die vorliegenden Untersuchungen wurde ein carbon-cage ohne Gitterstruktur verwendet, weil dieser mit zwei Kammern (I, II) verfügbar war, um einerseits den Wirkstoffkomplex und andererseits die autologe Spongiosa als Vergleich aufzunehmen.

Der eingesetzte Wirkstoffkomplex wurde aus Kälberknochen gewonnen, wie unter **I.** beschrieben und in eine Kammer (I) des carbon-cage eingebracht, während die andere Kammer (II) mit autologer Spongiosa des zu behandelnden Patienten befüllt wurde. Der so präparierte carbon-cage wird mittels eines cage-Setzgerätes im Bereich des Wirbelsäulensegmentes L5/S1 (Lendenwirbelsäule im Bereich der Bandscheiben) eingesetzt. Das den carbon-cage bereits aufweisende Setzgerät ist in Fig. 6 dargestellt, wobei in der Fig. die rechte Kammer (I) den Wirkstoffkomplex und die linke Kammer (II) autologe Spongiosa aufweist.

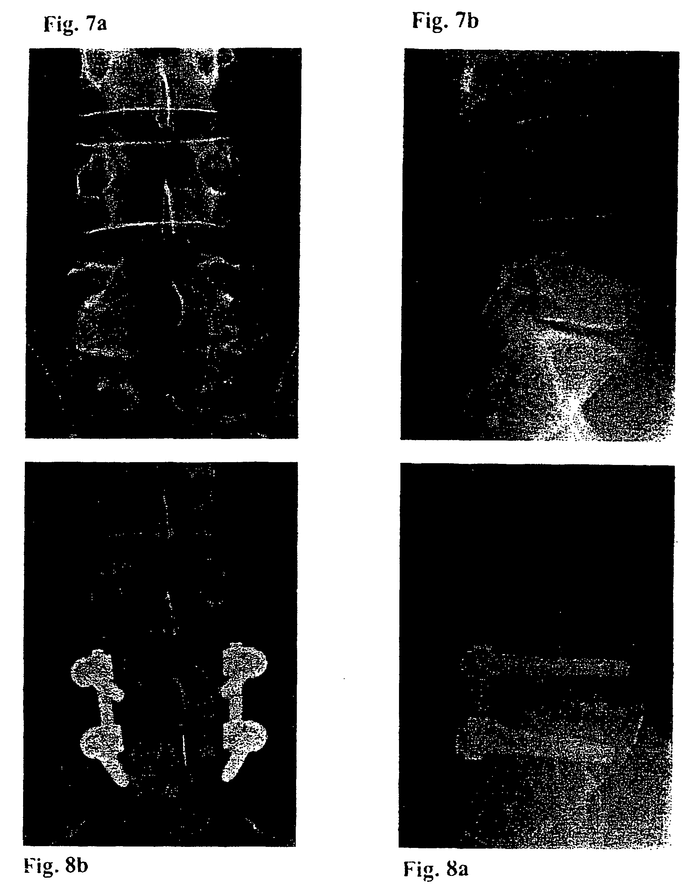

Die Fig. 7a, b zeigen den stark verringerten Zwischenwirbel-Abstand bei L5 und S1 vor dem Einsatz des Implantates.
Die Fig. 8 a, b zeigen die Abstützung durch das eingebrachte Implantat zwischen den Wirbeln L4 und L5 und den zur Stabiliserung eingebrachten "Fixateur intern".

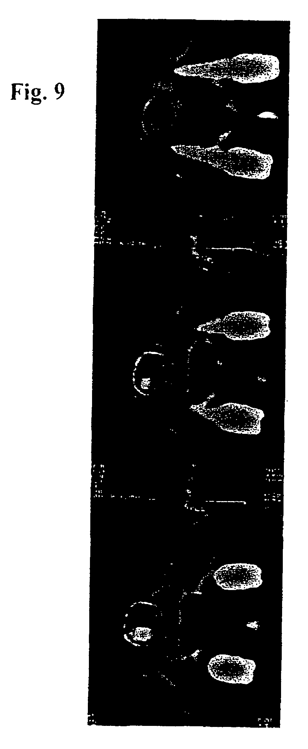

Fig. 9 zeigt -von links nach rechts gesehen- eine Abbildung der mittels Computertomograhie erhaltenen Bildsequenz jeweils drei, sechs und neun Wochen nach dem Einbringen des Käfigimplantates, wobei die linke Kammer des Käfigs das autologe Spongiosatransplantat und die rechte Kammer den Wirkstoffkomplex enthält. Man erkennt deutlich, daß in der linken Kammer mit Spongiosa die Röntgendichte als Zeichen des Knochenabbaus fortlaufend abnimmt, während sie in der rechten Kammer mit dem Wirkstoffkomplex als Zeichen der Knochenbildung über den gesamten Zeitraum zunimmt. Das erfindungsgemäße Implantat zeigt nach neun Wochen ein zumindest gleichwertiges Resultat durch Knochenaufbau wie das nach dem Stand der Technik als "Goldstandard" bewertete Autotransplantat durch Knochenabbau. Bei Verwendung des erfindungsgemäßen Implantats ist kein risikobehafteter Zweiteingriff erforderlich, und es wird keine Zeit für den primären Knochenabbau benötigt.

Tabelle 3 gibt die gemessene optische Dichte zu den in Fig. 9 graphisch gezeigten Untersuchungen wieder.

**Tabelle 3**

| Optische Dichte des mineralisierten Knochens im Implantat [%] | | | |
|---|---|---|---|
| | 3 Wochen | 6 Wochen | 12 Wochen |
| Autologe Spongiosa | 100 | 42 | 26 |
| Wirkstoffkomplex | 4 | 12 | 28 |

## Patentansprüche

1. Implantat für die zumindest teilweise Herstellung, Wiederherstellung oder Stabilisierung von Wirbelkörpern oder Röhrenknochen, bei dem ein metallischer, nichtmetallischer oder keramischer Hohlkörper mit einem Wirkstoffkomplex beschichtet ist oder einen Wirkstoffkomplex aufweist und dieser Wirkstoffkomplex die folgenden, voneinander unterschiedlichen, auf die Herzustellung von Knochen spezifisch abgestimmten Komponenten in Form mindestens einer Strukturkomponente auf der Basis von auf die Zellen des herzustellenden Knochens spezifisch abgestimmtem extrazellulärem Material, mindestens einer Rekrutierungskomponente, mindestens einer Adhäsionskomponente und mindestens einer Wachstums- und/oder Maturationskomponente aufweist.

2. Implantat nach Anspruch 1, **dadurch gekennzeichnet, daß** der metallische Hohlkörper aus Titan oder einer Titan-Legierung besteht.

3. Implantat nach Anspruch 1 oder 2, **dadurch gekennzeichnet, daß** der metallische Hohlkörper in Form eines Zylinders mit Gitterstruktur ausgestaltet ist.

4. Implantat nach Anspruch 1, **dadurch gekennzeichnet, daß** der nichtmetallische Hohlkörper aus Kohlenstoff-Fasern besteht.

5. Implantat nach Anspruch 1, **dadurch gekennzeichnet, daß** der keramische Hohlkörper aus einer Calciumphosphat-, Aluminiumoxid- oder Hydroxylapatitkeramik besteht.

6. Implantat nach einem der Ansprüche 1 bis 5, **dadurch gekennzeichnet, daß** der Hohlkörper mit dem Wirkstoffkomplex befüllt ist, wobei der Wirkstoffkomplex auf Trägermaterialien aufgebracht ist, die ausgewählt sind aus Polymeren und Collagenen.

## Claims

1. An implant for at least partially creating, recreating or stabilizing vertebral bodies or tubular bones, in which a metallic, nonmetallic or ceramic hollow body is coated with an active substance complex or comprises said active substance complex, and this active substance complex comprises the following components which differ from one another and which are specifically adapted for creating bone, namely at least one structural component based on extracellular material which is specifically adapted to the cells of the bone which is to be created, at least one recruiting component, at least one adhesion component, and at least one growth and/or maturation component.

2. An implant according to claim 1, **characterized in that** the metallic hollow body consists of titanium or a titanium alloy.

3. An implant according to claim 1 or 2, **characterized in that** the metallic hollow body is configured in the form of a cylinder with a lattice structure.

4. An implant according to claim 1, **characterized in that** the nonmetallic hollow body consists of carbon fibres.

5. An implant according to claim 1, **characterized in that** the ceramic hollow body consists of a calcium phosphate, aluminium oxide or hydroxylapatite ceramic.

6. An implant according to any one of claims 1 to 5, **characterized in that** the hollow body is filled with the active substance complex, said active substance complex being applied to support materials which are selected from polymers and collagens.

## Revendications

1. Implant pour la préparation au moins partielle, la reconstitution ou la stabilisation de vertèbres ou d'os tubulaires, dans lequel un corps creux métallique, non-métallique ou céramique est revêtu d'un complexe de matières actives ou présente un complexe de matières actives, lequel complexe de matières actives contient les composantes suivantes, différentes les unes des autres, convenant spécifiquement à la préparation de tissus osseux, et qui se présentent sous forme d'au moins une composante de structure à base d'un matériau extracellulaire spécifiquement adapté aux cellules osseuses, d'au moins une composante de recrutement, d'au moins une composante d'adhésion et d'au moins une composante de croissance et/ou de maturation.

2. Implant selon la revendication 1, **caractérisé par le fait que** le corps creux métallique est en titane ou en alliage de titane.

3. Implant selon la revendication 1 ou 2, **caractérisé par le fait que** le corps creux métallique a la forme d'une grille cylindrique.

4. Implant selon la revendication 1, **caractérisé par le fait que** le corps creux non-métallique est constitué de fibres de carbone.

5. Implant selon la revendication 1, **caractérisé par le fait que** le corps creux céramique est constitué d'une céramique à base de phosphate de calcium, d'oxyde d'aluminium ou d'hydroxyapatite.

6. Implant selon une des revendications 1 à 5, **caractérisé par le fait que** le corps creux est rempli avec le complexe de matières actives, le complexe de matières actives étant fixé sur des matériaux supports choisis parmi les polymères et le collagène.
